# EUROPEAN PATENT APPLICATION

(11) **EP 4 603 076 A1**
(43) Date of publication of application: **20.08.2025**
(21) Application number: 23877625.6
(22) Date of filing: 10.10.2023
(51) Int. Cl.: A61K 8/02, A61K 8/60, A61K 8/44, A61K 8/55, A61K 8/63, A61K 8/39, A61K 8/85, A61Q 19/00

(54) **LIPID NANOPARTICLE COMPOSITION COMPRISING SODIUM DNA**

(30) Priority: 11.10.2022 KR 20220129836
(71) Applicant: H&A Pharmachem Co., Ltd, Bucheon-si, Gyeonggi-do 14558 (KR)
(72) Inventor: JI, Hong Geun, Bucheon-si Gyeonggi-do 14600 (KR); PARK, Young Ah, Incheon 21069 (KR); KANG, Yu Jin, Seoul 08767 (KR)
(74) Representative: Schön, Christoph
(86) International application number: PCT/KR2023/015518
(87) International publication number: WO 2024/080698

(57) **Abstract**

The present invention relates to a lipid nanoparticle composition comprising sodium DNA, and more specifically relates to a lipid nanoparticle composition comprising: sodium DNA as an active ingredient; sodium dilauramidoglutamide lysine as an amphiphilic surfactant; distearoylphosphatidylcholine as an auxiliary surfactant; cholesterol as a stabilizer; an auxiliary stabilizer selected from PEG-120 stearate, polyglyceryl-4 caprate, a poly(ethylene glycol)-poly(ε-caprolactone) copolymer (PEG-PCL copolymer), and mixtures thereof; and a solvent.

## Description

### TECHNICAL FIELD

The present invention relates to a lipid nanoparticle composition comprising sodium DNA. More specifically, the present invention relates to a lipid nanoparticle composition comprising sodium DNA as an active ingredient; sodium dilauramidoglutamide lysine as an amphiphilic surfactant; distearoylphosphatidylcholine as an auxiliary surfactant; cholesterol as a stabilizer; an auxiliary stabilizer selected from PEG-120 stearate, polyglyceryl-4 caprate, poly(ethylene glycol)-poly(ε-caprolactone) copolymer (PEG-PCL copolymer) and a mixture thereof; and a solvent.

### BACKGROUND ART

Lipid nanoparticles (LNPs) are particulate nanocarriers composed of lipid membranes that can encapsulate insoluble or unstable substances. Lipid nanoparticles have attracted attention as an attractive delivery system due to their ease of manufacture, reduced immune response, high encapsulation efficiency, larger payload and design flexibility.

For example, the use of messenger ribonucleic acid (mRNA) to express therapeutic proteins has the potential to treat a variety of diseases. Therapeutic uses of mRNA are as follows: (1) protein replacement to restore single protein function in rare single-gene disorders; (2) cellular reprogramming in which mRNA can modulate cell behavior by expressing transcription or growth factors; (3) immunotherapy in which transcripts encoded by mRNA elicit specific immune responses to target cells, such as therapeutic antibodies, all of which are often hampered by inefficient nucleic acid delivery. The potential of lipid nanoparticles as carriers for mRNAs has been extensively studied. Lipid nanoparticles have many advantages over previous lipid-based delivery systems, including: i) high efficiency nucleic acid encapsulation and efficient transfection, ii) improved tissue permeability for therapeutic delivery and iii) low cytotoxicity and immunogenicity.

Meanwhile, sodium DNA (deoxyribonucleic acid) is a sodium salt of DNA. Sodium DNA is commonly called polydeoxyribonucleotide (PDRN) and a sodium salt of DNA extracted from the gonadic tissue of salmon and then purified. Sodium DNA is a tissue regeneration substance similar to human DNA, has high stability, and exists in cells to stimulate physiological regeneration and metabolic activity. Sodium DNA contributes to promoting growth factors, so it can be expected to have effects such as skin regeneration and inflammation relief, and thus it is attracting attention as a functional cosmetic ingredient. As an example of utilizing such sodium DNA, Korean Patent Application Publication No. 10-2020-0066420 discloses a skin anti-aging cosmetic composition comprising sodium DNA and astaxanthin as active ingredients.

### [Prior Document]

### [Patent Document]

Korean Patent Application Publication No. 10-2020-0066420

### DISCLOSURE OF INVENTION

### TECHNICAL PROBLEM

The technical problem of the present invention is the provision of a novel transdermal delivery system capable of efficiently transferring sodium DNA as an active substance through the skin while improving its stability, thereby increasing its bioavailability.

### SOLUTION TO PROBLEM

To solve the above technical problem, the present invention provides a lipid nanoparticle composition comprising sodium DNA as an active ingredient; sodium dilauramidoglutamide lysine as an amphiphilic surfactant; distearoylphosphatidylcholine as an auxiliary surfactant; cholesterol as a stabilizer; an auxiliary stabilizer selected from PEG-120 stearate, polyglyceryl-4 caprate, poly(ethylene glycol)-poly(ε-caprolactone) copolymer (PEG-PCL copolymer) and a mixture thereof; and a solvent.

In addition, the present invention provides a cosmetic composition comprising the lipid nanoparticle composition.

The present invention is described in detail hereinafter.

According to one aspect to the present invention, there is provided a lipid nanoparticle composition comprising sodium DNA as an active ingredient; sodium dilauramidoglutamide lysine as an amphiphilic surfactant; distearoylphosphatidylcholine as an auxiliary surfactant; cholesterol as a stabilizer; an auxiliary stabilizer selected from PEG-120 stearate, polyglyceryl-4 caprate, poly(ethylene glycol)-poly(ε-caprolactone) copolymer (PEG-PCL copolymer) and a mixture thereof; and a solvent.

The lipid nanoparticle composition of the present invention comprises sodium deoxyribonucleic acid (DNA) as an active ingredient. In one embodiment according to the present invention, the lipid nanoparticle composition may comprise sodium DNA as an active ingredient in an amount of 0.005 to 1% by weight, 0.01 to 0.5% by weight, or 0.04 to 0.1% by weight. In the present invention, if sodium DNA is comprised in an amount of less than 0.005% by weight, the effect according to the active ingredient may be weak, and if the amount of sodium DNA is more than 1% by weight, it may be problematic in the stability of the lipid nanoparticle.

The lipid nanoparticle composition of the present invention comprises sodium dilauramidoglutamide lysine as an amphiphilic surfactant. Sodium dilauramidoglutamide lysine is an amphiphilic surfactant having a tripeptide structure of glutamic acid-lysine-glutamic acid. In addition, sodium dilauramidoglutamide lysine has a gemini form having two or more hydrophilic groups and two or more hydrophobic groups, and has high solubility in water, low CMC (critical micelle concentration) and high ability of reducing surface tension. In another embodiment according to the present invention, the lipid nanoparticle composition may comprise sodium dilauramidoglutamide lysine as an amphiphilic surfactant in an amount of 0.001 to 2% by weight, 0.005 to 1% by weight, or 0.01 to 0.2% by weight. In the present invention, if sodium dilauramidoglutamide lysine is comprised in an amount less than 0.001% by weight, there may be a problem with the formation of the lipid nanoparticle, and if sodium dilauramidoglutamide lysine is comprised in an amount exceeding 2% by weight, there may be a problem with the stability of the lipid nanoparticle.

The lipid nanoparticle composition of the present invention comprises distearoylphosphatidylcholine (1,2-distearoyl-sn-glycero-3-phosphocholine, DSPC) as an auxiliary surfactant. Distearoylphosphatidylcholine is phosphatidylcholine, a type of phospholipid, which is a natural component of cell membranes. In another embodiment according to the present invention, the lipid nanoparticle composition may comprise distearoylphosphatidylcholine as an auxiliary surfactant in an amount of 0.0001 to 1% by weight, 0.0005 to 0.5% by weight, or 0.001 to 0.1% by weight. In the present invention, if distearoylphosphatidylcholine is comprised in an amount of less than 0.0001 % by weight, there may be a problem with the formation of the lipid nanoparticle, and if distearoylphosphatidylcholine is comprised in an amount exceeding 1 % by weight, there may be a problem with the stability of the lipid nanoparticle.

The lipid nanoparticle composition of the present invention comprises cholesterol as a stabilizer. In the present invention, cholesterol plays a role in enhancing the stability of the lipid membrane structure. In another embodiment according to the present invention, the lipid nanoparticle composition may comprise cholesterol as a stabilizer in an amount of 0.001 to 2% by weight, 0.005 to 1% by weight, or 0.01 to 0.2% by weight. In the present invention, if cholesterol is comprised in an amount of less than 0.001% by weight, the stabilizing effect by cholesterol may be weak, and if cholesterol is comprised in an amount exceeding 2% by weight, there may be a problem in the formation of the lipid nanoparticle.

The lipid nanoparticle composition of the present invention comprises, as an auxiliary stabilizer, one selected from PEG-120 stearate, polyglyceryl-4 caprate, poly(ethylene glycol)-poly(ε-caprolactone) copolymer (PEG-PCL copolymer) and a mixture thereof. PEG-120 stearate is a polyethylene glycol ester of glyceryl stearate. Polyglyceryl-4 caprate is an ester of capric acid and polyglycerin-4. Poly(ethylene glycol)-poly(ε-caprolactone) copolymer (PEG-PCL copolymer) is an amphiphilic block copolymer that can be prepared by polymerizing polyethylene glycol and polycaprolactone, and is biodegradable. In another embodiment according to the present invention, the auxiliary stabilizer may be a mixture of PEG-120 stearate and polyglyceryl-4 caprate. In another embodiment according to the present invention, the lipid nanoparticle composition may comprise the auxiliary stabilizer in an amount of 0.0001 to 1% by weight, 0.0003 to 0.5% by weight, or 0.0005 to 0.1% by weight. In the present invention, if the auxiliary stabilizer is comprised in an amount of less than 0.0001% by weight or more than 1% by weight, there may be a problem in the formation of the lipid nanoparticle.

The lipid nanoparticle composition of the present invention comprises a solvent. In another embodiment according to the present invention, the solvent may be selected from alcohol, water and a mixture thereof. In another embodiment according to the present invention, the solvent may be a mixture of alcohol and water. In another embodiment according to the present invention, the alcohol may be ethanol. In another embodiment according to the present invention, the lipid nanoparticle composition may comprise the solvent in an amount of 93 to 99.99% by weight, 96.5 to 99.95% by weight or 99.3 to 99.9% by weight. In the present invention, if the solvent is comprised in an amount of less than 93% by weight or more than 99.99 % by weight, there may be a problem in the formation of the lipid nanoparticle.

In another embodiment according to the present invention, the lipid nanoparticle composition can be prepared by a thin-film hydration method, a reverse phase evaporation method, an emulsion method or a microfluidics. In another embodiment according to the present invention, the lipid nanoparticle composition may be prepared by a microfluidics. Microfluidics can prepare a lipid-based nano-carriers using an organic solvent such as ethanol and a phospholipid in a microfluidic chip. Microfluidics can control the particle size and the membrane surface by adjusting the production process conditions such as the speed of the fluid, the type and content of the lipid, etc. Microfluidics can have advantages of being a continuous process and having high reproducibility.

According to another aspect of the present invention, there is provided a cosmetic composition comprising the lipid nanoparticle composition. In another embodiment according to the present invention, the cosmetic composition may comprise the lipid nanoparticle composition in an amount of 1 to 50% by weight. In the present invention, if the cosmetic composition comprises the lipid nanoparticle composition in an amount of less than 1% by weight, the effect of sodium DNA as the active ingredient may be weak, and if the amount of the lipid nanoparticle composition is more than 50% by weight, it may be economically undesirable since increasing the effect according to the active ingredient commensurately with the added amount would not be expected.

### EFFECTS OF INVENTION

According to the present invention, by formulating the active ingredient, sodium DNA, into lipid nanoparticles, the sodium DNA can be efficiently delivered into the skin in a very stable state, thereby exhibiting high bioavailability, and also providing excellent effects on skin regeneration and inflammation relief.

### BRIEF DESCRIPTION OF DRAWINGS

Figure 1A is a photograph of the appearances of lipid nanoparticles prepared in Example 1, and Figure 1B is a photograph of the appearances of lipid nanoparticles prepared in Example 2.
Figure 2A is a result of measuring the particle distribution of lipid nanoparticles prepared in Example 1, and Figure 2B is a result of measuring the particle distribution of lipid nanoparticles prepared in Example 2.
Figure 3A is a result of measuring the zeta potential of lipid nanoparticles prepared in Example 1, and Figure 3B is a result of measuring the zeta potential of lipid nanoparticles prepared in Example 2.
Figure 4A is a result of measuring the Fourier transform infrared spectroscopy (FT-IR) spectrum of lipid nanoparticles prepared in Example 1, and Figure 4B is a result of measuring the Fourier transform infrared spectroscopy (FT-IR) spectrum of lipid nanoparticles prepared in Example 2.
Figure 5Ais a result of measuring the high temperature stability of L1W3, L1W4, L1W5 and L1W6 samples of Example 1 using Turbiscan, and Figure 5B is a result of measuring the high temperature stability of L1W4(D), L1W5(D), L1W6(D) and L1W7(D) samples of Example 2 using Turbiscan.
Figure 6 is a result of measuring the ultraviolet absorption spectrum of lipid nanoparticles prepared in Example 2.
Figure 7 is an enlarged photograph of lipid nanoparticles prepared in Example 2 using a freeze-fracture scanning electron microscopy (× 12,000).

### MODE FOR THE INVENTION

### Example 1: Preparation of lipid nanoparticles not containing active ingredient (L1W1 - L1W10)

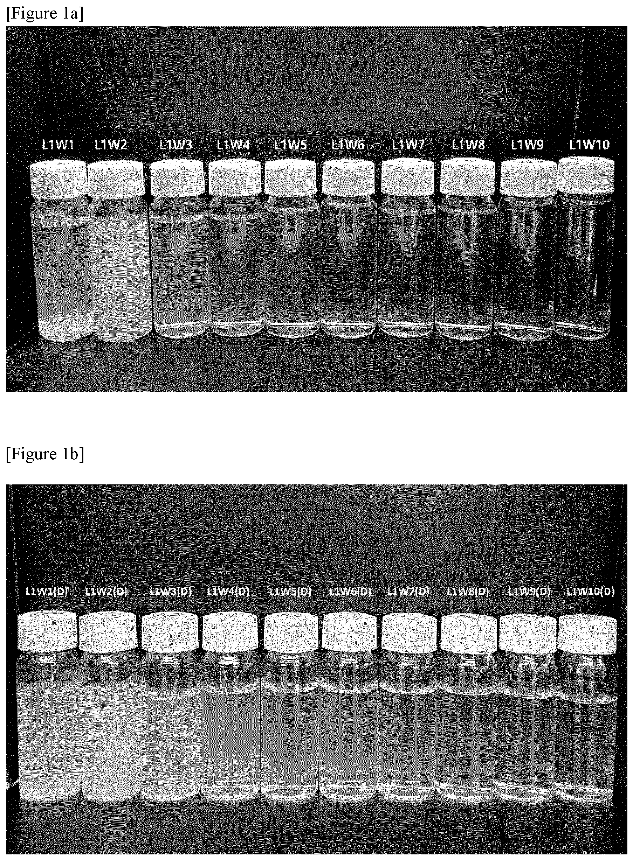

First of all, a lipid solution and an aqueous solution were prepared separately. Lipid nanoparticles were prepared using a microfluidic device (Liposome Synthesis System, NEO NANOTECH, Korea), and the device can control the rpm of the driving motor through the feedback function between the flow sensor and the driving motor. The previously prepared lipid solution and aqueous solution were placed in the microfluidic chip, and the lipid nanoparticles of L1W1 to L1W10 were prepared by adjusting the flow rate with the compositions in Table 1 below. At this time, the flow rate can be controlled from a minimum of 0.0015 mL/min to a maximum of 18 mL/min. The appearances of the prepared lipid nanoparticles were photographed and represented in Figure 1A.

**[Table 1] Preparation of lipid solution and aqueous solution**

| | Ingredient | Content (% by weight) | | | | |
|---|---|---|---|---|---|---|
| | | L1W1 | L1W2 | L1W3 | L1W4 | L1W5 |
| Lipid Solution | Sodium Dilauramidoglutamide Lysine | 0.125 | 0.080 | 0.063 | 0.050 | 0.042 |
| | Cholesterol | 0.075 | 0.05 | 0.037 | 0.03 | 0.025 |
| | DSPC (Distearoylphosphatidylcholine) | 0.025 | 0.017 | 0.012 | 0.010 | 0.008 |
| | PEG-120 Stearate/Polyglyceryl-4 Caprate | 0.005 | 0.003 | 0.003 | 0.002 | 0.002 |
| | Ethanol | 49.770 | 33.180 | 24.885 | 19.908 | 16.590 |
| Aqueous Solution | Purified water | 50.000 | 66.670 | 75.000 | 80.000 | 83.333 |

| | Ingredient | Content (% by weight) | | | | |
|---|---|---|---|---|---|---|
| | | L1W6 | L1W7 | L1W8 | LlW9 | L1W10 |
| Lipid Solution | Sodium Dilauramidoglutamide Lysine | 0.036 | 0.031 | 0.027 | 0.025 | 0.023 |
| | Cholesterol | 0.021 | 0.019 | 0.017 | 0.015 | 0.013 |
| | DSPC (Distearoylphosphatidylcholine) | 0.007 | 0.006 | 0.006 | 0.005 | 0.004 |
| | PEG-120 Stearate/Polyglyceryl-4 Caprate | 0.002 | 0.001 | 0.001 | 0.001 | 0.001 |
| | Ethanol | 14.22 | 12.443 | 11.06 | 9.954 | 9.049 |
| Aqueous Solution | Purified water | 85.714 | 87.500 | 88.889 | 90.000 | 90.910 |

### Example 2: Preparation of lipid nanoparticles containing sodium DNA [L1W1(D) - L1W 10(D)]

A lipid solution and an aqueous solution were prepared separately. Lipid nanoparticles were manufactured using the same microfluidic device as in Example 1. In the microchip, the flow rate of each solution was adjusted to prepare lipid nanoparticles of L1W1(D) to L1W10(D) with the compositions in Table 2 below. The appearances of the prepared lipid nanoparticles were photographed and represented in Figure 1B.

**[Table 2]**

| Ingredient | | Content (% by weight) | | | | |
|---|---|---|---|---|---|---|
| | | L1W1(D) | L1W2(D) | L1W3(D) | L1W4(D) | L1W5(D) |
| Lipid Solution | Sodium Dilauramidoglutamide Lysine | 0.125 | 0.080 | 0.063 | 0.050 | 0.042 |
| | Cholesterol | 0.075 | 0.05 | 0.037 | 0.03 | 0.025 |
| | DSPC (Distearoylphosphatidylcholine) | 0.025 | 0.017 | 0.012 | 0.010 | 0.008 |
| | PEG-120 Stearate/Polyglyceryl-4 Caprate | 0.005 | 0.003 | 0.003 | 0.002 | 0.002 |
| | Ethanol | 49.770 | 33.180 | 24.885 | 19.908 | 16.590 |
| Aqueous Solution | Purified water | 49.95 | 66.603 | 74.925 | 79.92 | 83.25 |
| | Sodium DNA | 0.05 | 0.067 | 0.075 | 0.08 | 0.083 |

| | Ingredient | Content (% by weight) | | | | |
|---|---|---|---|---|---|---|
| | | L1W6(D) | L1W7(D) | L1W8(D) | L1W9(D) | L1W10(D) |
| Lipid Solution | Sodium Dilauramidoglutamide Lysine | 0.036 | 0.031 | 0.027 | 0.025 | 0.023 |
| | Cholesterol | 0.021 | 0.019 | 0.017 | 0.015 | 0.013 |
| | DSPC (Distearoylphosphatidylcholine) | 0.007 | 0.006 | 0.006 | 0.005 | 0.004 |
| | PEG-120 Stearate/Polyglyceryl-4 Caprate | 0.002 | 0.001 | 0.001 | 0.001 | 0.001 |
| | Ethanol | 14.22 | 12.443 | 11.06 | 9.954 | 9.049 |
| Aqueous Solution | Purified water | 85.628 | 87.412 | 88.800 | 89.910 | 90.819 |
| | Sodium DNA | 0.086 | 0.088 | 0.089 | 0.090 | 0.091 |

### Experimental Example 1: Measurement of Particle Size Distribution

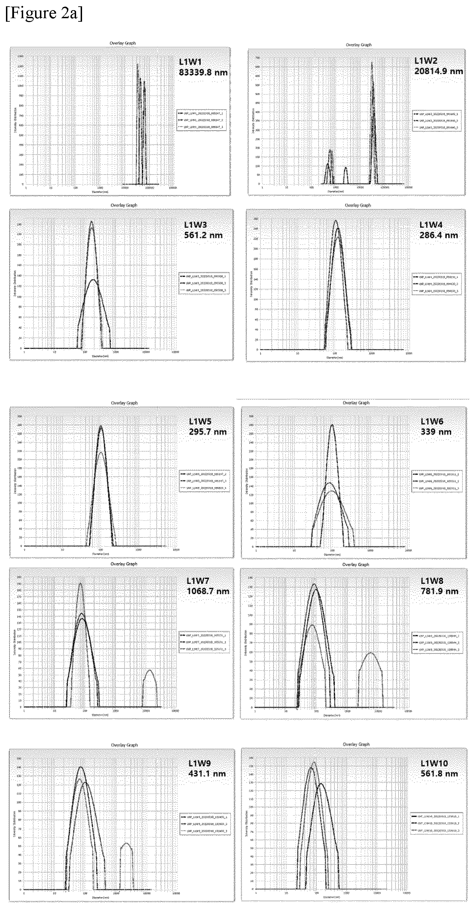

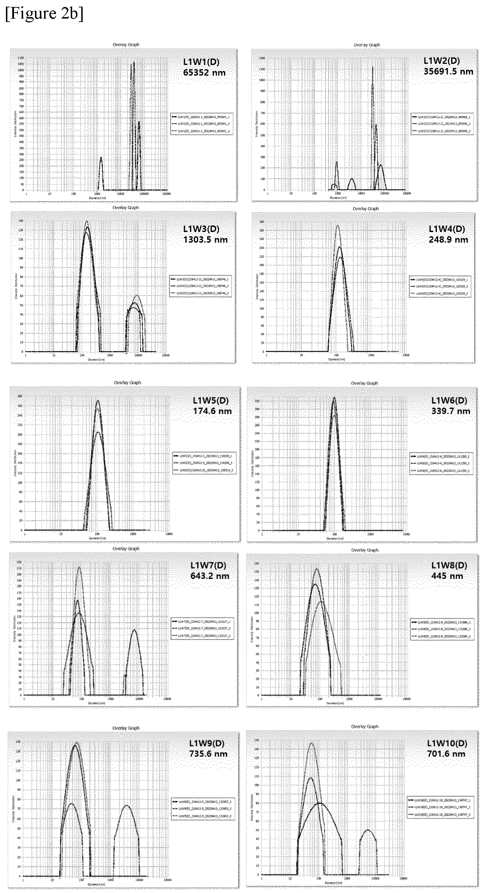

In order to ascertain the particle size distribution of the lipid nanoparticles prepared in Examples 1 and 2, the lipid nanoparticle solution was diluted by 10%, and 10 mL was taken into a Photal, ELS-Z (Photal, Japan) cell and measured. The results are represented in Figures 2A and 2B. As can be seen in Figures 2A and 2B, it can be known that the particle size distribution differed depending on the difference in composition by the adjustment of the flow rate.

### Experimental Example 2: Measurement of Zeta Potential

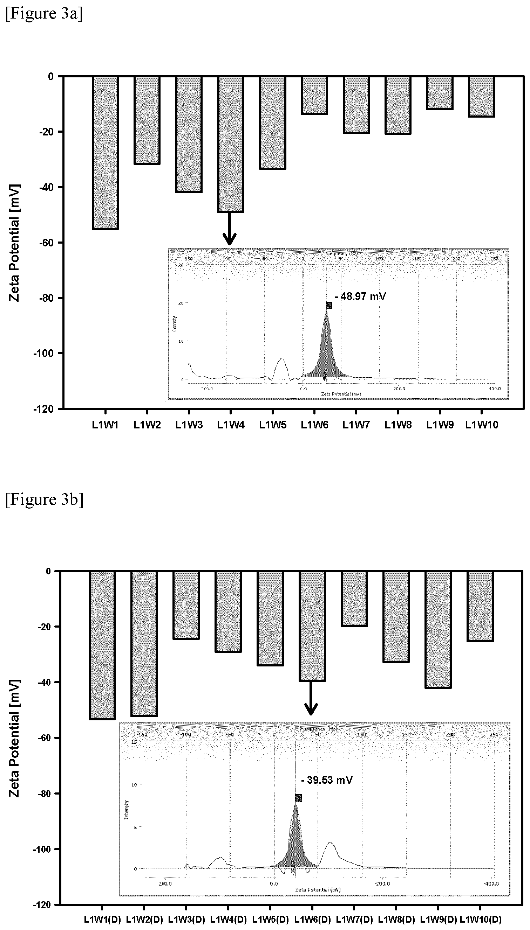

In order to ascertain the surface charge of the lipid nanoparticles prepared in Examples 1 and 2, the lipid nanoparticle solution was diluted by 10%, 1 mL was taken into a Photal, ELS-Z (Photal, Japan) cell, and the zeta potential was measured. The results are represented in Figures 3A and 3B. As can be seen in Figures 3A and 3B, it can be known that the surface charge of the particles differed depending on the difference in composition by the adjustment of the flow rate.

### Experimental Example 3: Fourier-transform infrared (FT-IR) spectroscopy

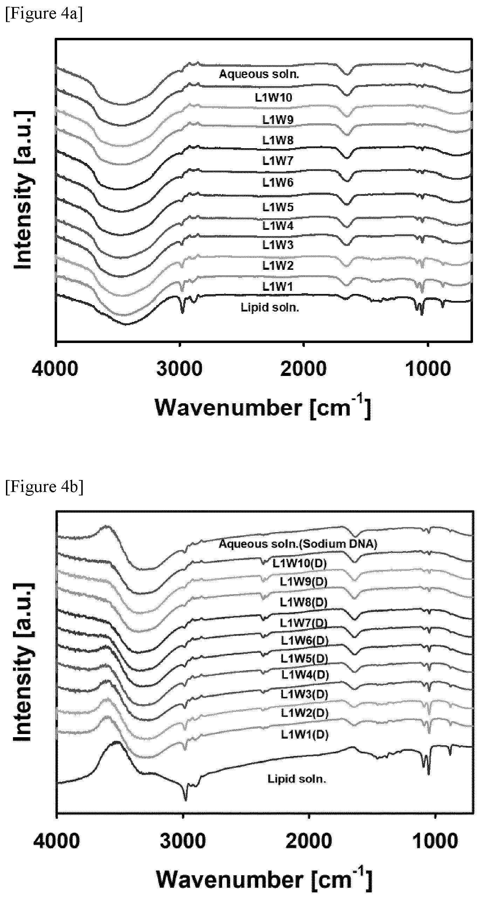

In order to ascertain the composition of the lipid nanoparticles prepared in Examples 1 and 2, FT-IR was measured by using a Nicolet Summit FTIR spectrometer (ThermoFisherScientific, USA), and the results are represented in Figures 4A and 4B. It was confirmed that as the ratio of the aqueous solution increased, the lipid-related peak decreased and the aqueous-related peak increased.

### Experimental Example 4: Turbiscan measurement

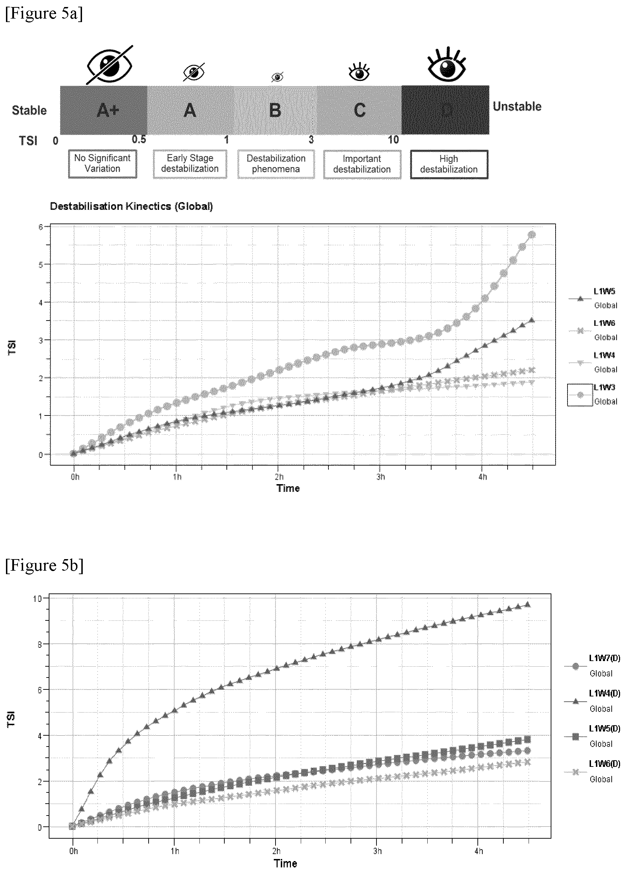

In order to ascertain the high-temperature stability of the L1W3, L1W4, L1W5, and L1W6 samples, which are relatively stable in particle size and zeta potential among the lipid nanoparticles prepared in Example 1, measurements were performed using TurbiscanLAB (Formulaction, France), and the results are represented in Figure 5A. The analysis conditions were the temperature of 40°C and 50 scans for 5 hours. It was confirmed that L1W4 is the most stable In Example 1. In the lipid nanoparticles prepared in Example 2, the high-temperature stability of the L1W4(D), L1W5(D), L1W6(D) and L1W7(D) samples was measured, and the results are represented in Figure 5B. It was confirmed that L1W6(D) is the most stable in Example 2.

### Experimental Example 5: Measurement of UV absorption spectrum

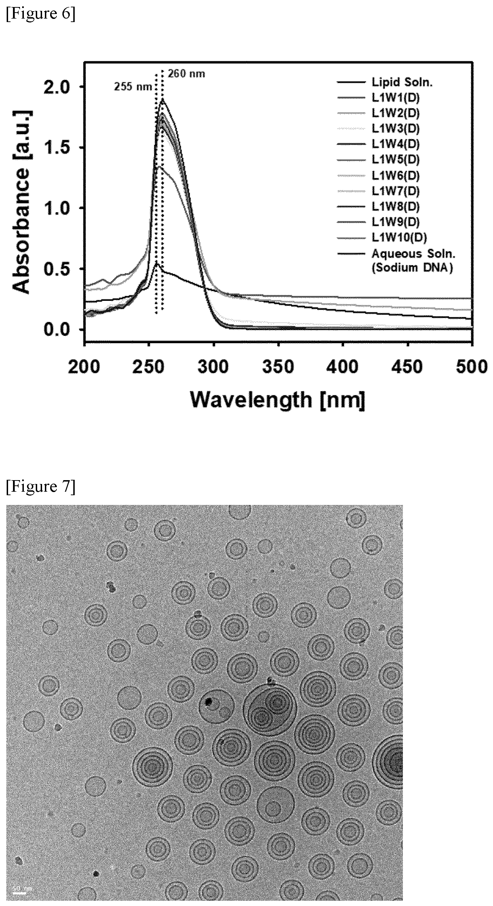

In order to ascertain whether the lipid nanoparticles prepared in Example 2 contain sodium DNA, the absorption spectrum was measured by using a UV Spectrometer (ThermoFisher Scientific, USA), and the results are represented in Figure 6. As the aqueous solution ratio increased, it could be seen that the sodium DNA peak was generated and the peak shift occurred.

### Experimental Example 6: Freezing electron microscopy

The sodium DNA-lipid nanoparticles manufactured in Example 2 were photographed at a magnification of × 12,000 using a freezing electron microscope (JEM 1010, JEOL, Japan) (Figure 7). From Figure 7, it can be known that the lipid nanoparticles were well formed with a uniform size.

## Claims

1. A lipid nanoparticle composition comprising sodium DNA as an active ingredient; sodium dilauramidoglutamide lysine as an amphiphilic surfactant; distearoylphosphatidylcholine as an auxiliary surfactant; cholesterol as a stabilizer; an auxiliary stabilizer selected from PEG-120 stearate, polyglyceryl-4 caprate, poly(ethylene glycol)-poly(ε-caprolactone) copolymer (PEG-PCL copolymer) and a mixture thereof; and a solvent.

2. The lipid nanoparticle composition according to Claim 1, which comprises 0.005 to 1% by weight of sodium DNA, 0.001 to 2% by weight of sodium dilauramidoglutamide lysine, 0.0001 to 1% by weight of distearoylphosphatidylcholine, 0.001 to 2% by weight of cholesterol, 0.0001 to 1% by weight of the auxiliary stabilizer and 93 to 99.99% by weight of the solvent.

3. The lipid nanoparticle composition according to Claim 2, which comprises 0.01 to 0.5% by weight of sodium DNA, 0.005 to 1% by weight of sodium dilauramidoglutamide lysine, 0.0005 to 0.5% by weight of distearoylphosphatidylcholine, 0.005 to 1% by weight of cholesterol, 0.0003 to 0.5% by weight of the auxiliary stabilizer and 96.5 to 99.95% by weight of the solvent.

4. The lipid nanoparticle composition according to Claim 3, which comprises 0.04 to 0.1% by weight of sodium DNA, 0.01 to 0.2% by weight of sodium dilauramidoglutamide lysine, 0.001 to 0.1% by weight of distearoylphosphatidylcholine, 0.01 to 0.2% by weight of cholesterol, 0.0005 to 0.1% by weight of the auxiliary stabilizer and 99.3 to 99.9% by weight of the solvent.

5. The lipid nanoparticle composition according to Claim 1, which is prepared by a thin-film hydration method, a reverse phase evaporation method, an emulsion method or microfluidics.

6. The lipid nanoparticle composition according to Claim 5, which is prepared by microfluidics.

7. The lipid nanoparticle composition according to Claim 1, the auxiliary stabilizer is a mixture of PEG-120 stearate and polyglyceryl-4 caprate.

8. The lipid nanoparticle composition according to Claim 1, wherein the solvent is selected from alcohol, water and a mixture thereof.

9. The lipid nanoparticle composition according to Claim 8, wherein the alcohol is ethanol.

10. A cosmetic composition comprising a lipid nanoparticle composition as defined in any one of Claims 1 to 9.

11. The cosmetic composition according to Claim 10, which comprises 1 to 50% by weight of the lipid nanoparticle composition.
